# EUROPEAN PATENT APPLICATION

(11) **EP 4 122 600 A1**
(43) Date of publication of application: **25.01.2023**
(21) Application number: 21187525.7
(22) Date of filing: 23.07.2021
(51) Int. Cl.: B01L 3/00, B01L 7/00

(54) **APPARATUS AND ELECTRONIC DEVICE FOR ANALYSING SAMPLES**

(71) Applicant: Nokia Technologies Oy, 02610 Espoo (FI)
(72) Inventor: KIM, John, Murray Hill, 07974 (US); ZHENG, Mingde, New Providence, 07974 (US)
(74) Representative: Swindell & Pearson Limited

(57) **Abstract**

Examples of the disclosure relate to an apparatus for analysing fluid samples. The apparatus is sized and shaped so that it can fit into an input port of an electronic device. The input port could be an existing port of the electronic device such as an input port for a memory card or a charger. The electronic device can be configured with a heat transfer means so that, when the apparatus is inserted into the electronic device, heat from the electronic device can be used to control the temperature of a fluid sample within the apparatus. This can enable the reaction conditions within the apparatus to be controlled.

## Description

### TECHNOLOGICAL FIELD

Examples of the disclosure relate to an apparatus and electronic device for analysing samples. Some relate to an apparatus and electronic device for analysing samples where the apparatus can be removably inserted into an input port of the electronic device to enable analysis of the sample.

### BACKGROUND

Fluid samples can be analysed to determine the presence of particular chemicals, molecules, biological species or analytes. However, the reactions and the sample processing can require specific conditions. It is imperative to enable these conditions to be provided within a device that is convenient to use.

### BRIEF SUMMARY

According to various, but not necessarily all, examples of the disclosure there is provided an apparatus:
wherein the apparatus is configured to be removably inserted into an input port of an electronic device and wherein the apparatus comprises:
at least one reaction chamber configured to receive a fluid sample and enable the fluid sample to come into contact with one or more reagents;
at least one sensor for sensing a reaction between the fluid sample and the one or more reagents; and
temperature control means configured to use heat from the electronic device to control a temperature of the fluid sample in the at least one reaction chamber.

The at least one sensor for sensing a reaction may comprise at least one functionalized electrode.

The at least one functionalized electrode may be configured to be user replaceable.

The temperature control means may comprise one or more sensors for determining the temperature of the at least one reaction chamber and means for providing a control signal to control heat provided from the electronic device to the at least one reaction chamber based upon the determined temperature.

The temperature control means may comprise one or more sensors for determining the temperature of the reaction chamber wherein the one or more sensors are also configured to provide a control signal to control heat provided to the reaction chamber based upon the determined temperature.

The apparatus may comprise means for providing an output indicative of the presence and concentration of an analyte within the fluid sample.

The apparatus may comprise an output device configured to provide an output indicative of the presence and concentration of an analyte within a sample.

The apparatus may comprise one or more data connections configured to enable data to be transmitted from the apparatus to the electronic device.

The apparatus may comprise means for receiving the fluid sample and means for enabling the fluid sample to flow from the means for receiving the fluid sample into at least one reaction chamber.

The means for receiving the fluid sample and means for enabling the fluid sample to flow from the means for receiving the fluid may be configured to enable fluid to flow into the reaction chamber without requiring a user input.

The means for receiving the fluid sample and means for enabling the fluid sample to flow from the means for receiving the fluid may be configured to enable fluid to flow into the reaction chamber in response to a user input.

The apparatus may comprise a receptacle for receiving the fluid sample and a conduit for enabling the fluid sample to flow from the receptacle into at least one reaction chamber.

The receptacle and conduit may be configured to enable fluid to flow into the reaction chamber without requiring a user input.

The receptacle may be configured to enable fluid to flow into the reaction chamber in response to a user input.

The at least one reaction chamber may comprises one or more elongated microfluidic channel and one or more functionalized electrodes.

The elongate microfluidic channel may comprise one or more meandering structures.

According to various, but not necessarily all, examples of the disclosure there is provided an apparatus:
wherein the apparatus is configured to be removably inserted into an input port of an electronic device and wherein the apparatus comprises:
at least one reaction chamber configured to receive a fluid sample and enable the fluid sample to come into contact with one or more reagents;
at least one sensor for sensing a reaction between the fluid sample and the one or more reagents; and
at least one temperature controller configured to use heat from the electronic device to control a temperature of the fluid sample in the at least one reaction chamber.

According to various, but not necessarily all, examples of the disclosure there is provided an electronic device comprising:
one or more heat sources;
one or more input ports configured to enable an apparatus for analysing a fluid sample to be removably inserted into the input port; and
heat transfer means configured to transfer heat from the one or more heat sources to a location proximate to the one or more input ports.

The heat transfer means may comprise at least one of: a heat pipe, thermosyphon loop, oscillating heat pipe.

The heat transfer means may comprise a thermal interface in the one or more input ports configured to enable heat to be transferred to an apparatus for analysing a fluid sample when the apparatus for analysing a fluid sample is removably inserted into the input port.

The electronic device may comprise one or more data connections configured to enable data to be transmitted between the electronic device and the apparatus for analysing a fluid sample.

The one or more heat sources may comprise at least one of: a battery, electronic components, optoelectronic components, processors.

At least one of the one or more input ports may be configured to receive a plurality of different input components.

The electronic device may comprise a hand-held communications device.

According to various, but not necessarily all, examples of the disclosure there is provided an electronic device comprising:
one or more heat sources;
one or more input ports configured to enable an apparatus for analysing a fluid sample to be removably inserted into the input port; and
at least one heat transfer system configured to transfer heat from the one or more heat sources to a location proximate to the input ports.

### BRIEF DESCRIPTION

Some examples will now be described with reference to the accompanying drawings in which:
Fig. 1 shows an example apparatus and electronic device;
Figs. 2A to 2D show example electronic devices and heat transfer means;
Fig. 3 shows an example apparatus and a section of an electronic device;
Fig. 4 shows an example system manager:
Figs. 5A to 5C show an example receptacle and conduit;
Figs. 6A and 6B show another example receptacle;
Fig. 7 shows an example reaction or processing apparatus;
Figs. 8A to 8D show example electrodes;
Fig. 9 shows an example electrode and apparatus: and
Figs. 10A and 10B show an example electrode in use.

### DETAILED DESCRIPTION

Examples of the disclosure relate to an apparatus for analysing fluid samples. The apparatus is sized and shaped so that it can fit into an input port of an electronic device. The input port could be an existing port of the electronic device such as an input port for a memory card or a charger. The electronic device can be configured with a heat transfer means so that, when the apparatus is inserted into the electronic device, heat from the electronic device can be used to control the temperature of a fluid sample within the apparatus. This can enable the reaction conditions within the apparatus to be controlled.

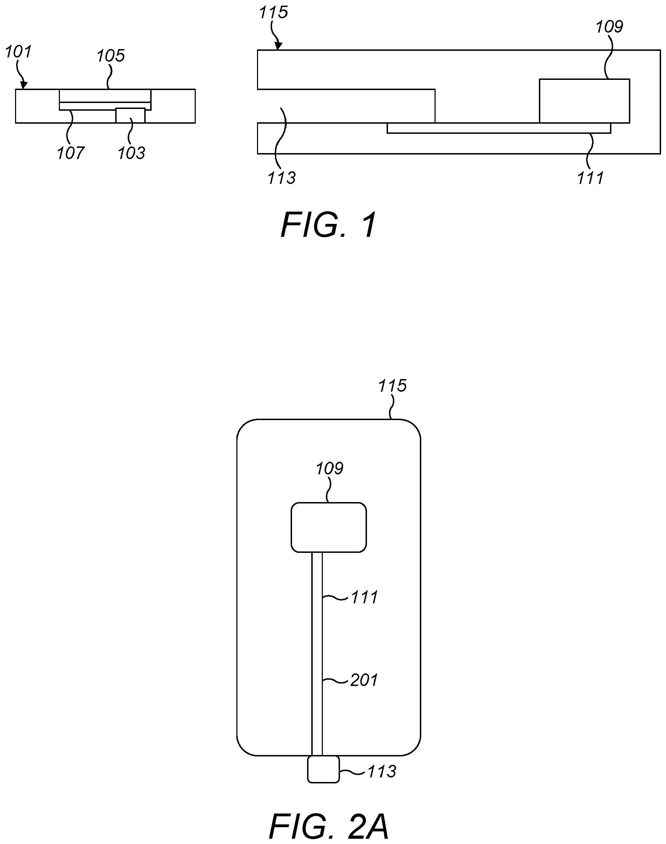

Fig. 1 schematically shows an example apparatus 101 and electronic device 115 according to examples of the disclosure. These are not shown to scale in Fig. 1. Some examples of the disclosure can comprise features that are not shown in Fig. 1. For example, the electronic device 115 can comprise processors and transceivers to enable wireless communication and/or any other suitable components. The apparatus 101 can comprise additional features as described below. These features could be provided in any suitable combination.

The electronic device 115 can be a handheld communications device such as a mobile phone, tablet computer or any other suitable type of device. The electronic device 115 can be configured to be functional without the apparatus 101 being inserted. For example, the electronic device 115 can be used to make and receive communications without the apparatus 101 being inserted.

The example electronic device 115 shown in Fig. 1 comprises a heat source 109, heat transfer means 111 and at least one input port 113.

The heat source 109 can comprise any components of the electronic device that generate heat during use. The heat source 109 can comprise components that generate unwanted heat during use. For example, the heat source 109 could comprise one or more electronic components such as processing units or graphical processing units (GPU) and/or a battery or any other suitable type of components. These components can generate unwanted heat and can require cooling to maintain efficiency.

In some examples the heat source 109 can comprise a resistive element or other type of heating component. The resistive element can be configured to generate heat for the apparatus 101 when needed. For instance, if the apparatus 101 is not inserted within the electronic device 115 then the resistive element could be inactive so that no heat is generated. The resistive element could be activated in response to an indication that the apparatus 101 has been inserted in the input port 113. The amount of heat generated by the resistive element could be controlled to control the temperature of the apparatus 101. The resistive element could be provided in any suitable location within the electronic device 115.

The heat transfer means 111 can comprise any means for transferring heat from the heat source 109 to a location proximate to the input port 113.

In some examples the heat transfer means 111 can comprise a heat pipe, a thermosyphon loop, an oscillating heat pipe, an electroresistive heat pipe, or any other suitable heat transfer means 111. In some examples an insulating material can be provided around the heat transfer means 111 in the sections between the heat source 109 and the input port 113 so as to prevent heat being dissipated at locations other than the input port 113.

The heat transfer means 111 can be configured to provide localised heating of the input port 113 so that the heat generated by the heat source 109 can be used to control the temperature of the apparatus 101 and the fluid sample within the apparatus 101.

The input port 113 comprises a cavity within the housing of the electronic device 115. The cavity is sized and shaped so as to receive an input device and enable the input device to be coupled to the electronic device 115. The input port 113 can be configured to receive any suitable type of input device, for example the input port 113 can be configured to receive a charging device, a memory card, a USB (universal serial bus) device, a headphone jack or any other suitable type of input.

As well as being sized and shaped to receive a corresponding input device, the input port 113 can also be configured to enable signals to be exchanged between the electronic device 115 and the input device. For instance, in some examples the input port 113 could comprise a cavity for receiving a charging connector. In such examples the input port 113 can comprise electrical connections to enable power to be transferred from the charging connector to a power source of the electronic device 115. In some examples the input port 113 can comprise a cavity for receiving a memory card or other suitable inputs. In such examples the input port 113 can comprise electrical connections to enable data to be transferred between the memory card the electronic device 115.

In the example of Fig.1 only one heat source 109 is shown. It is to be appreciated that in other examples of the disclosure the electronic device 115 could comprise a plurality of different heat sources 109. For example, the electronic device 115 could comprise one or more processors and also a battery and/or a resistive heating element. Different heat sources 109 can be located in different positions within the electronic device 109. In some examples the heat sources 109 can comprises component that draw power from the electronic device 115. For example, the electronic device 115 could be designed so that the processors 115 are not located close to other sources of heat. In such examples the heat transfer means 111 can be configured to transfer heat from the plurality of different locations of the heat sources 109 to the location proximate to the input port 113.

The apparatus 101 is configured to fit into the input port 113 of the electronic device 115. The apparatus 101 can have a size and shape so that it can fit into the input ports of the electronic device 115. The apparatus 101 can have a size and shape so that it can fit securely into the input ports of the electronic device 115. In some examples the apparatus 101 could comprise one or more connectors to enable the apparatus 101 to be electrically connected to the electronic device 115. The connectors could be positioned on the apparatus 101 so that they connect to corresponding connectors in the electronic device 101 when the apparatus 101 is inserted into the receiving port 113. This can enable data to be exchanged between the electronic device 115 and the apparatus 101.

The apparatus 101 comprises a reaction chamber 105, a sensor 107 and temperature control means 103. The apparatus 101 could comprise additional components that are not shown in Fig. 1.

The reaction chamber 105 can comprise any means that is configured to receive a fluid sample and enable the fluid sample to come into contact with one or more reagents. In some examples the reaction chamber 105 could comprise a recess on the surface of the apparatus 101. The recess of the reaction chamber 105 can be sized and shaped so as to maximise, or substantially maximise, the volume of fluid that comes into contact with the reagent.

In some examples the reagents could be provided on the sensor 107. For example, the sensor 107 could be comprise a functionalised electrode where the reagents are coated on the surface of the electrode. In such examples the sensor 107 could be provided as part of a wall of the reaction chamber 105 so that when fluid is contained within the reaction chamber 105 chamber the fluid sample is in contact with the sensor 107. This can enable a reaction to take place between the reagent and the fluid sample. Whether or not a reaction occurs can depend upon the analytes present within the fluid sample and the reagents provided within the sensor 107.

Other types of sensors 107 could be used in other examples of the disclosure. For instance, in some examples the sensor 107 could comprise a thermometer that can be configured to act as a calorimeter to monitor the chemical reactions occurring within the reaction chamber 105. The thermometer could be provided within the reaction chamber 105 or in any other suitable location. In some examples the thermometer could be provided on the electronic device rather than the apparatus 101. For instance, the thermometer could be provided within the input port 113 of the electronic device 115.

The sensor 107 can detect the reaction and provide an output indicative of the reaction. This therefore enables an output to be provided where the output indicates whether or not a particular analyte is present or its concentration within the fluid sample. This enables an output to be provided indicative of a reaction occurring within the reaction chamber 105.

The apparatus 101 also comprises temperature control means 103. The temperature control means 103 can comprise any means that can be configured to control the temperature of the fluid sample in the reaction chamber 105. The temperature control means 103 can be configured to use heat transferred by the heat transfer means 111 from the heat source 109 of the electronic device 115 to the location proximate to the input port 113 to heat a fluid sample, or at least part of a fluid sample, in the reaction chamber 105. In some examples the temperature control means 103 can be configured to use heat transferred by the heat transfer means 111 from the heat source 109 of the electronic device 115 to the location proximate to the input port 113 to maintain a constant, or substantially constant temperature, of the fluid sample in the reaction chamber 105.

In some examples the temperature control means 103 can comprise a thermal interface that is configured to enable efficient heat transfer from the heat transfer means to the fluid sample. The thermal interface can comprise a thermally conductive material such as a metal.

In some examples the temperature control means 103 can comprise one or more temperature sensors. The temperature sensors can be configured to monitor the temperature of the fluid in the reaction chamber 105 or the temperature proximate to the reaction chamber 105 so as to enable a constant, or substantially constant temperature to be maintained.

Therefore, examples of the disclosure enable unwanted heat from an electronic device 115 to be used to control the temperature of a reaction of an apparatus 101. In some examples the heat can be used to maintain the fluid sample at a constant, or substantially constant temperature. This can enable the apparatus 101 to be used for reactions that require specific temperature conditions. This can enable the reactions to be performed using the apparatus 101 and the electronic device 115 rather than requiring the reaction to be performed in a laboratory or other specific environment. As the electronic device could be a mobile phone or other similar device this can make it easier for the samples to be analysed because a user can use the apparatus 101 with their own existing electronic device 115.

Figs. 2A to 2D show example electronic devices 115 and heat transfer means 111 according to some examples of the disclosure.

Fig. 2A shows an example electronic device 115. The electronic device 115 comprises a heat source 109, heat transfer means 111 and an input port 113.In the example of Fig. 2A the heat transfer means can comprise a heat pipe 201.

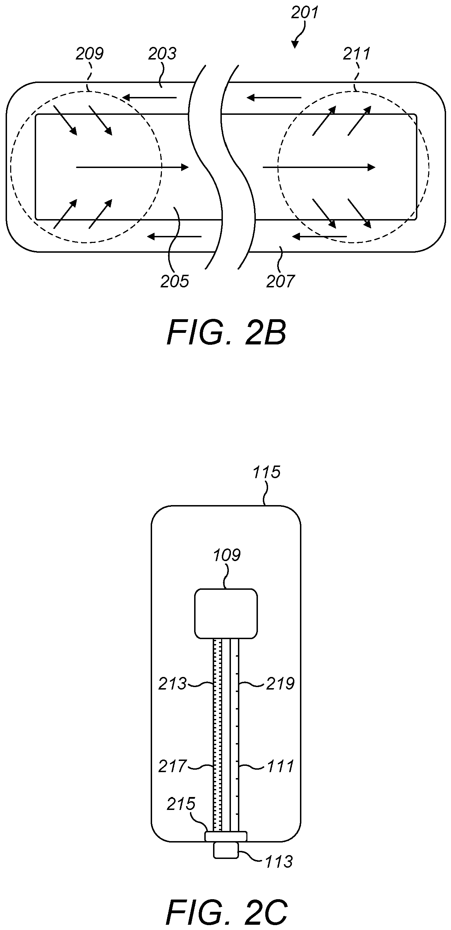

Fig. 2B schematically illustrates a cross section example heat pipe 201 that could be used as a heat transfer means 111 in the example of Fig. 2A. The heat pipe 201 provides an example of a two-phase cooling system that can be used. The heat pipe 201 comprises three layers an upper layer 203, a gas channel 205 and a lower layer 207. The upper layer 203 and the lower layer 207 provide a wick structure around the gas channel 205. The wick structure comprises a plurality of capillary channels that enable liquid to be transported along the upper layer 203 and the lower layer 207.

A working fluid is provided within the heat pipe 201. The working fluid could be water or any other suitable fluid. When the heat pipe 201 is in use the working fluid circulates through the gas channel 205 and the upper layer 203 and the lower layer 207 so as transfer heat from the heat source 109 to the location proximate the input port 113. When the working fluid is in the gas channel 205 the working fluid is in a gas phase and when the working fluid is in the upper layer 203 or the lower layer 207 the working fluid is in a liquid phase.

A first end of the heat pipe 201 provides an evaporator region 209 in a location close to the heat source 109. A second end of the heat pipe 201 provides a condenser region 211 in a location proximate the input port 113.

At the evaporator region 209, the heat from the heat source 109 causes the working fluid to evaporate and change phase from a liquid to a gas. The working fluid in the gas phase travels from the evaporator region 209 to the condenser region 211. At the condenser region 211, the comparatively cooler temperature causes the working fluid to condense and change phase from a gas to a liquid. As result, heat is moved from the evaporator region 209 to the condenser region 211.

At the condenser region 211, the working fluid condenses back into a liquid phase and travels back to the evaporator region 209 through capillary action of the wick structure in the upper layer 203 and the lower layer 207. Once the liquid phase working fluid has reached the evaporator region 209 again the heat at the evaporator region 209 will change the working fluid back into the gas phase. The cycle of the working fluid changing phase repeats so as to drive the working fluid in the gas phase and the heat from the evaporator region 209 to the condenser region 211.

In some examples the section of the heat pipe 201 between the evaporator region 209 and the condenser region 211 can be insulated. The insulation may help to prevent heat being lost through the heat pipe 201.

In the example of Figs. 2A and 2B the heat pipe 201 comprises a first end and a second end where the evaporator region 209 is located at the first end and the condenser region 211 is located at the second end. In other examples the heat pipe 201 could comprise a plurality of branches. For instance, electronic device 115 could comprise a plurality of heat sources 109 and different evaporator regions 209 could be provided proximate to the different heat sources 109. Similarly, the electronic device 115 could comprise a plurality of different input ports 113 and different condenser regions 211 could be provide proximate to the different input ports 113.

Fig. 2C shows an example electronic device 115. The electronic device 115 comprises a heat source 109, heat transfer means 111 and an input port 113.In the example of Fig. 2C the heat transfer means can comprise a thermosyphon loop 213. The thermosyphon loop 213 is another two-phase cooling system that can be used in examples of the disclosure.

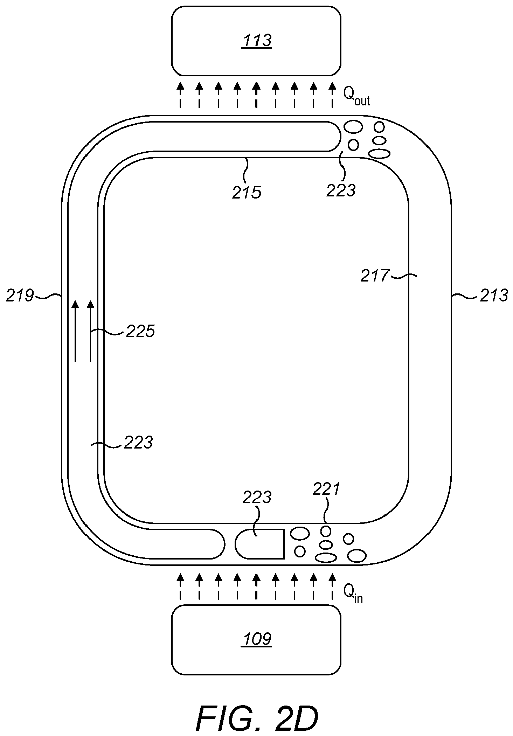

Fig. 2D shows a schematic example two-phase cooling system 101 that can be used in examples of the disclosure. The thermosyphon loop 213 shown in Fig. 2D comprises an evaporator 221, a condenser 215, a downcomer 217 and a riser 219. A working fluid 223 is provided within the thermosyphon loop 213. When the thermosyphon loop 213 is in use the working fluid 223 circulates through the components of the thermosyphon loop 213.

The evaporator 221 is provided at the bottom of the thermosyphon loop 213 so that the working fluid 223 flows down the downcomer 217 into the evaporator 215 under the force of gravity. The evaporator 221 comprises any means for transferring heat from a heat source 109 into the working fluid 223. The evaporator 221 is thermally coupled to the heat source 109.

Heat is transferred from the heat source 109 to the working fluid 223 in the evaporator 221. This heat transfer causes a partial evaporation of working fluid 223 within the evaporator 221 and converts the working fluid 223 from a liquid phase into a mixture of liquid and vapour phase. The two-phase mixture can comprise droplets of vapour entrained within the liquid, liquid slugs and vapor plugs or other flow regimes depending on the design of the thermosyphon loop 213, heat load, filling ratio, working fluid and any other suitable parameter.

The evaporator 221 is coupled to the riser 219 so that the working fluid expelled from the evaporator 221 flows into the riser 219. This working fluid 223 comprises a two-phase mixture where the vapour phase is less dense than the liquid phase. The working fluid 223 within the thermosyphon loop 213 rises through the riser 219, as indicated by the arrows 225. The passive flow in the thermosyphon loop 213 is driven by the density difference between the working fluid 223 in the liquid phase in the downcomer 217 and the working fluid 223 in the two-phase mixture in the riser 219.

The condenser 215 is provided at the top of the thermosyphon loop 213. The condenser 215 is positioned above the evaporator 223 so that the working fluid 223 flows upwards from the evaporator 221 to the condenser 215. In other examples the thermosyphon loop 213 could comprise a pump to drive the circulation of the working fluid 223.

The condenser 215 is coupled to the riser 219 so that the working fluid 223 in the two-phase mixture flows from the riser 219 into the condenser 215. The condenser 255 can comprise any means for cooling the working fluid 223. The condenser can be positioned proximate to the input port 113 so as to enable heat from the working fluid 223 to be transferred to an apparatus 101 within the input port 113. This heat transfer causes the working fluid 223 to condense, at least partly, back into the liquid phase.

The condenser 215 is coupled to the downcomer 217 so that the working fluid 223 can flow down the downcomer 217 by gravity and be returned to the inlet of the evaporator 223 to start the cycle again.

Figs. 2A to 2D show example electronic devices 115 and heat transfer means. 111. Other types of heat transfer means 111 can be used in other examples of the disclosure. For instance, the heat transfer means 111 can be another type of two-phase cooling system such as an oscillating heat pipe. In other examples the heat transfer means 111 could be a single-phase cooling system.

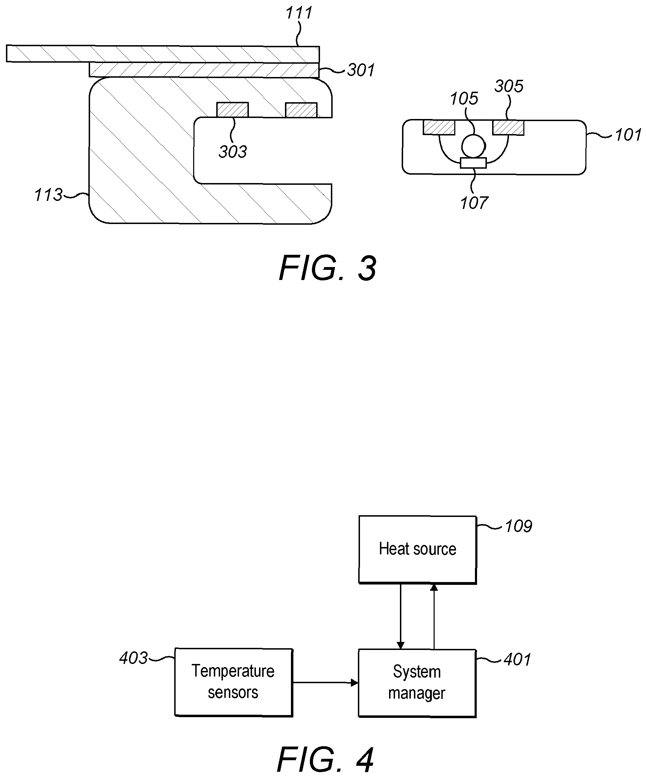

Fig. 3 shows another example apparatus 101 and section of an electronic device 115. Fig. 3 shows the input port 113 and the heat transfer means 111. The heat transfer means 111 can comprise a heat pipe 201, a thermosyphon loop 213 or any other suitable type of heat transfer means 111.

A thermal interface material 301 is provided between the heat transfer means 111 and the input port 113. The thermal interface material 301 can be provided in a thin layer so as to optimize, or substantially optimize, heat transfer from the heat transfer means 111 to the input port 113.

The input port 113 can comprise electrical connections 303. The electrical connections 303 can enable data to be transferred between the electronic device 115 and the apparatus 101 when the apparatus 101 is positioned within the input port 113. In some examples the electrical connections 303 can also be thermally conductive which can enable heat to be transferred to the apparatus 101.

In some examples the input port 113 can be retrofitted so as to receive the apparatus 101. For instance, a plug can be inserted into the input port 113 so that the apparatus 101 can be received tightly into the input port 113. The plug can comprise metal or any other thermally conductive material so as to enable heat to be transferred from the heat transfer means to the apparatus 101.

The apparatus 101 can comprise a chip or card. The apparatus 101 is sized and shapes so as to fit into the input port 113. The apparatus 101 can be small, for example the apparatus 101 could have a surface area that is smaller than around 1 cm² or any other suitable size. The size and shape of the apparatus 101 can be determined by the size and shape of the input port 113 of the electronic device 115 or by any other factor.

The apparatus 101 is configured to be removably inserted into the input port 113 of the electronic device 115. This can enable a user to insert the apparatus 101 into the input port 113 and also enable the user to remove the apparatus 101 from the input port 113 after the sample has been analysed. The apparatus 101 and the electronic device 115 can be configured to enable a user to insert and remove the apparatus 101 into the input port 113 without using any tools or special equipment.

The apparatus 101 comprises a reaction chamber 105. The reaction chamber 105 can be configured to receive a fluid sample. The fluid sample could comprise a biological sample such as bodily fluids from a patient. In some examples the fluid sample could comprise an environmental sample, for instance a sample of water or other fluid collected from an environment to be tested.

The reaction chamber 105 can be configured to enable a user to insert the fluid into the reaction chamber 105.

The reaction chamber 105 can comprise one or more reagents. The reagents can comprise chemicals that are selected to react with a particular analyte within a sample. The reagents can comprise bio-chemical reagents or any other suitable type of reagents. In some examples the reagents could comprise one or more biomarkers that are configured to bind with or otherwise interact with, one or more analytes. The reaction of the reagents with any analytes in the sample therefore provides an indication of the presence and/or concentration of the analyte within the sample. The type of reagent that is used depends upon the types of samples that the apparatus 101 is intended to analyse, the type of analyte that the apparatus 101 is intended to sense and/or any other factor.

In some examples the reagents can be provided within the reaction chamber 105. In some examples the reagent can be provided on a functionalized electrode. For example, a biomarker, or other reagent, can be coated on the surface of an electrode. This can enable the functionalized electrode to provide a sensor 107 for sensing the presence of the analyte.

The sensor 107 can comprise any means for sensing a reaction between the fluid sample and the one or more reagents. The sensor 107 can be configured to sense the chemical reaction in real time as the reaction occurs. In some examples the sensor can comprise a functionalised electrode. Other types of electrode or sensor 107 could be used in other examples of the disclosure.

The sensor 307 can be coupled to the electrical connections 305. The electrical connections 305 are configured to couple to the corresponding contacts 303 in the input port 113. This can enable data and other activating or de-activating signals to be exchanged between the electronic device 115 and the apparatus 101. For example, it can enable information indicative of the reaction occurring in the reaction chamber to be transmitted from the sensor 107 in the apparatus 101 to the electronic device 115.

When the apparatus 101 is in use, a user inserts a fluid sample into the reaction chamber 105. The fluid sample can comprise bodily fluids, environmental fluids or any other type of fluid. The user then inserts the apparatus 101 comprising the fluid sample into the input port 113 of the electronic device 115. When the apparatus 101 is inserted within the input port 113, heat from the heat transfer means 111 can be used to heat the fluid sample. The heat can be used to heat the fluid sample to a temperature that enables a reaction to occur between analytes in the fluid sample and the reagents in the reaction chamber 105. In some examples the heat can be applied to enable stable chemical reactions to take place or to maintain stability of analytes, reagents and/or fluid samples.

The sensor 107 senses whether or not one or more analytes are present within the sample based on the reaction between the fluid sample and the reagent. The sensor 107 can then provide an output signal indicative of whether or not the one or more analytes are present and/or their corresponding concentrations. The output signal can be transmitted from the apparatus 101 to the electronic device 115 via the contacts 305, 303 or via any other suitable means. The sensor 107 and the contacts 303, 305 can therefore provide means for providing an output indicative of a reaction occurring in the reaction chamber. Other means for providing the output could be used in other examples of the disclosure, for instance, the output does not need to be an electrical signal.

The electronic device 115 can receive the signal from the apparatus 101 and, based on that signal, provide an output to a user indicative of whether or not the one or more analytes are present in the fluid sample. For example, information could be displayed on a display of the electronic device 115, or information could be provided using any other suitable means.

The heat from the heat source 109 of the electronic device 115 can therefore be used to initiate or speed up the reaction between the fluid sample and the reagents in the reaction chamber 105. In some examples the apparatus 101 and/or electronic device 115 can be configured to enable the temperature of the fluid to be maintained within a specific temperature range. This can enable the apparatus 101 to be used for reactions which require specific temperature conditions. For instance, a polymerase chain reaction (PCR) can be performed at a constant temperature of around 65°C depending upon the propriety enzymes that are used. Such reactions could be performed using the example apparatus 101 and heat from the heat source 109 to enable target RNA or DNA sequences to be detected.

Also, when the apparatus 101 is not in use and inserted in the input port 113 the heat transfer means 111 can still be used to transfer heat away from the heat source 109. This can allow for efficient cooling of electronic components within the electronic device 115.

In some examples the apparatus 101 can comprises a system control manager. The system control manager can be configured to control the temperature of the fluid within the reaction chamber 105. The system control manager can provide temperature control means for controlling and maintaining the temperature of the apparatus 101. In some examples the apparatus 101 can also comprise one or more temperature sensors configured to determine the temperature of the reaction chamber 105. The temperature sensors can also be configured to provide an output signal indicative of the determined temperature. The temperature sensors therefore provide means for providing a control signal to control heat provided to the reaction chamber 105 based upon the determined temperature.

Fig. 4 shows an example system manager 401 that could be used in some examples of the disclosure. The system manager 401 could be configured to control the temperature of the reaction chamber 105 or to control the temperature of any other suitable part of the apparatus 101. The system manager 401 could be positioned on the apparatus 101 or in the electronic device 115.

The system manager 401 is configured to receive an input signal from one or more temperature sensors 403. The temperature sensors 403 could be provided in the reaction chamber 105 or in a location proximate to the reaction chamber 105. This can enable the temperature conditions within the reaction chamber 105 to be determined.

The system manager 401 is also configured to receive an input from the heat source 109 of the electronic device 115. The input can indicate the heat being generated by the heat source 109 of the electronic device 115. For example, if the heat source 109 is a processor the system manager 401 can receive an input indicative of the current workload of the processor. In examples where the heat source 109 comprises a resistive heating element the input can indicate the current passing through the resistive element.

The system manager 401 uses the received temperature information to determine the heat needed from the heat transfer means 111. For example, if the temperature sensor 403 indicates that the apparatus 101 is below a threshold temperature then further heating from the heat source 109 and heat transfer means 111 is needed. Similarly, if the temperature sensor 403 indicates that the apparatus 101 is at or above a threshold temperature then no further heating is needed from the heat transfer means 111.

The system manager 401 is configured to provide an output signal to the heat source 109 to adjust the heat generated by the heat source 109 and control the amount of heat provided to the apparatus 101. For example, if the heat source 109 is a processor the heat generated by the heat source 109 can be adjusted by adjusting the work load of the heat source 109. In examples where the heat source 109 comprises a resistive heating element the heat generated by the heat source 109 can be adjusted by adjusting the current passing through the resistive element. Other means for adjusting the heat generated by the heat sources 109 and/or provided to the apparatus 101 can be used in other examples of the disclosure.

The system manager 401 therefore allows for dynamic control of the temperature of the apparatus 101. This can help to maintain correct temperature conditions for specific reactions and so enables the apparatus 101 to be used for a wide range of chemical reactions.

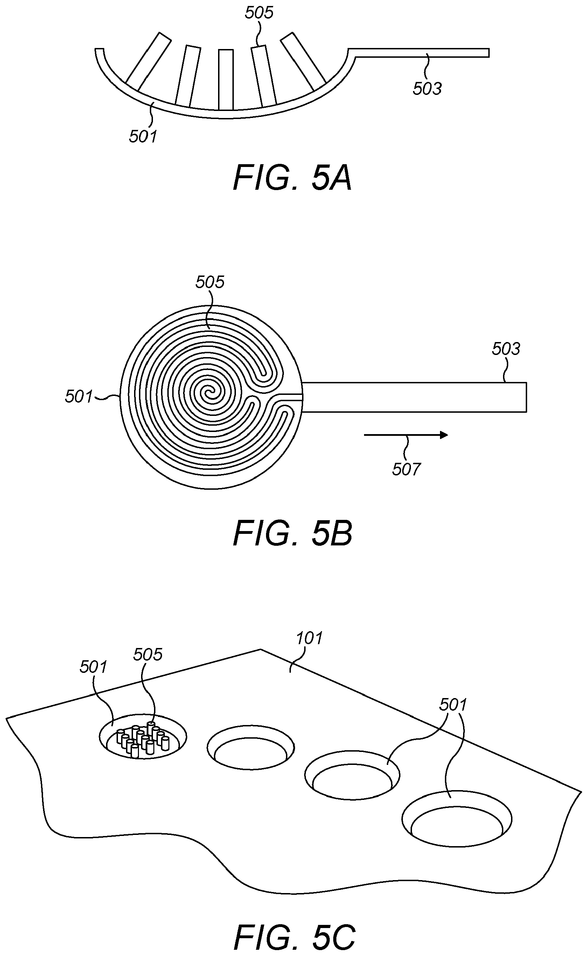

Figs. 5A to 5C show an example receptacle 501 and conduit 503. The receptacle and conduit 503 are configured to receive a fluid sample and enable the fluid sample to be provided to a reaction chamber. Fig. 5A shows a side view of the receptacle 501 and conduit 503 and Fig. 5B shows a plan view of the receptacle 501 and conduit 503. Fig. 5C shows some example receptacles on the surface of the apparatus 101. The conduits 503 are not shown in Fig. 5C.

The receptacle 501 comprises a recess within the surface of the apparatus 101. The recess is deep enough to enable a fluid to be retained within the receptacle 501. In the example of Figs. 5A to 5C the receptacle 501 is circular. This avoids any sharp corners which would reduce the mobility of a fluid sample moving through the receptacle 501 and conduit 503.

The size of the receptacle 501 can be determined by the size of the apparatus 101, the fluid to be provided within the apparatus 101 and/or any other suitable factors. In some examples the receptacle 501 could have a diameter in the range of 1 to 10mm, or substantially within 1 to 10mm. The depth of the receptacle 501 could be between 50 to 500micrometres or substantially between 50 to 500micrometres. Other sizes for the receptacle 501 could be used in other examples of the disclosure.

The conduit 503 extends between the receptacle 501 and the reaction chamber 105 of the apparatus 101. The reaction chamber 105 is shown not shown in Figs. 5A to 5C. The conduit 503 is configured to enable the fluid sample to flow from the receptacle 501 to the reaction chamber 105 as indicated by the arrow 507 in Fig. 5B.

In the example of Figs. 5A to 5C the conduit 501 is configured to enable the fluid to flow passively from the receptacle 501 to the reaction chamber 105. The fluid flows passively in that the receptacle 501 and conduit 503 are configured to enable fluid to flow into the reaction chamber 105 without requiring a user input.

In the example of Figs. 5A to 5C a single conduit 503 is provided for the receptacle 501 and single outlet 507 is provided from the receptacle 501 into the conduit 503. Om other examples a plurality of conduits can be connected to the receptacle 501 with any number of outlets 507 leading to the reaction chamber 105.

In the example of Figs. 5A to 5C the receptacle 501 comprises a plurality of walls 505. The walls 505 are provided across the surface of the receptacle 501 and can be configured to enable capillary action of the fluid sample out of the receptacle and into the conduit 503. The walls 505 can be provided in a dense arrangement and with a high aspect ratio so as to enable the capillary action. The walls 505 maximise, or substantially maximise, the amount of fluid retained within the receptacle 501 and directed towards the reaction chamber 105. The walls 505 help to prevent excess sample fluid from spilling over into adjacent receptacles 501 which could reduce the amount of fluid available for analysis and/or contaminate the samples within the other receptacles 501.

The walls 505 can be provided in a maze-like structure on the surface of the receptacle 501 as shown in Fig. 5B. Fig. 5C shows a section of the walls 505. These walls 505 could be provided as continuous unbroken structures in examples of the disclosure. In Fig. 5B the walls have a circular, or substantially, circular shape, other shapes and arrangements of the walls 505 could be used in other examples.

Fig. 5C shows a plurality of different receptacles 501 provided on the surface of the substrate 101. The different receptacles 501 can be used to receive different fluid samples or enable a plurality of samples of the same fluid to be received in the apparatus 101.

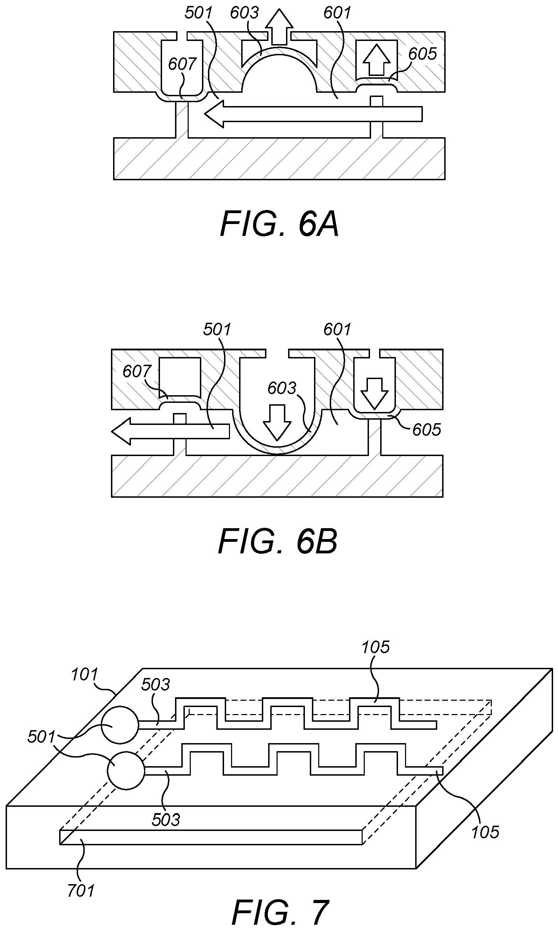

Figs. 6A and 6B show another example receptacle 501 according to examples of the disclosure. In this example the the receptacle 501 is configured to enable fluid to flow into the reaction chamber 105 in response to a user input. This can enable the user to control when the fluid flows into the reaction chamber 105. This can enable a user to control when any reactions between the fluid sample and the reagents begin. This can also enable the fluid to flow into the reaction chamber 105 more quickly which could make the apparatus 101 more convenient for a user to use.

In the example of Figs. 6A and 6B the receptacle 501 comprises an actuation chamber 601. The actuation chamber 601 comprises a cavity that can be configured to receive a fluid sample and contain the fluid sample until a user input is made.

The actuation chamber 601 comprises a user input device 603, a first valve 605 and a second valve 607. The actuation chamber 601 could comprise other components in other examples of the disclosure.

The user input device 603 comprises means for enabling a user to control the flow of fluid out of the actuation chamber 603. The user input device 603 can comprise a button or any other suitable means that can be actuated by a user of the apparatus 101. The user input device 603 can be actuated by a user applying a force to the user input device 603 by pushing the user input device 603 or by any other suitable means.

In the example of Figs. 6A and 6B the user input device 603 comprises a flexible member that is biased to be in a configuration in which the flexible member is curved upwards when the user input device 603 is not actuated. The user can actuate the user input device 603 by applying a force so that the flexible member is curved downwards. Other configurations for the user input device 603 could be used in other examples.

The flexible member of the user input device 603 can be made from a water impermeable material or from a material that is substantially water impermeable. The material that is used can be dependent upon the type of fluid that is used for the fluid sample or for any other factors.

The first valve 605 can be configured to control flow of a fluid sample into the actuation chamber 603. The second valve 607 can be configured to control the flow of the fluid sample out of the actuation chamber 601. In the example of Figs. 6A and 6B the first valve 605 and the second valve 607 are provided on opposing sides of the actuation chamber 603. The first valve 605 is provided on a first side of the user input device 603 and the second valve 607 is provided on a second side of the user input device 603. This arrangement can enable the fluid sample to flow into the actuation chamber 603 in a first side and then flow out of the actuation chamber 603 at the second side.

Fig. 6A shows the user input device 603 in an unactuated state. In this state the flexible member is curved upwards and the first valve 605 is open. This enables the fluid sample to be received into the actuation chamber 601.

Fig. 6B shows the user input device 603 in an actuated state. In this state a user has pushed the user input device 603 or otherwise applied a force to the user input device 603 so that the user input device 603 is curved downwards. The actuation of the user input device 603 causes the first valve 605 to close and second valve 607 to open. This causes the fluid sample to flow through the second valve 607 out of the actuation chamber 601 due to hydrostatic forces. The second valve 607 can be coupled to the conduit 503 so that the fluid flows out of the actuation chamber 601, through the second valve 607 and towards the reaction chamber 105. This therefore enables the fluid sample to flow out of the actuation chamber 603 in response to a user input. Once the fluid flows out of the actuation chamber 601 the fluid can flow along the conduit 503 to the reaction chamber 105 based on momentum and/or capillary action.

Once the fluid sample has flown out of the actuation chamber 601 the force can be removed from the user input device 603 and the user input device 603 can return to the unactuated configuration shown in Fig. 6A. This can then enable another fluid sample to be added to the apparatus.

The user input device 603 can enable a user to quickly input the fluid sample into the reaction chamber 105. This can help to prevent accumulation or overflowing of the fluid sample. In some examples this can also allow for a plurality of different samples to be added to the actuation chamber 603. This could also allow for a cleaning fluid to be washed through the apparatus to allow for washing of the apparatus 101 and/or the removal of debris from the apparatus 101.

Fig. 7 schematically shows an example apparatus 101. The example apparatus 101 comprises a plurality of reaction chambers 105 and an aperture 701 for receiving an electrode. The electrode can provide a sensor 107 configured to detect reactions between a reagent and fluid sample within the reaction chambers 105.

Each of the reaction chambers 105 is fluidically coupled to a receptacle 501 and a conduit 503. The receptacle 501 and conduit 503 could be as shown in Figs. 5A to 5C or in any other configuration. In the example of Fig. 7 the different reaction chambers 105 are coupled to different receptacles 501. This can enable different fluid samples to be provided to the different reaction chambers.

In the example of Fig. 7 the reaction chambers 105 comprise an elongated microfluidic channel. The reaction chambers 105 can have a high aspect ratio so as to enable the wicking of the fluid to be effective. For example, the aspect ratio could be 1 to 100, or any other suitable value. In some examples, different sections of the reaction chambers 105 can have different aspect ratios. The different aspect ratios can control the flow rate of the fluid though the reaction chambers 105. Areas with a reduced aspect ratio can be used to slow down the fluid flow due to capillary action and areas with an increased aspect ratio can be used to speed up the fluid flow due to capillary action. The slower flow of fluid can be used in area where an extended chemical reaction time is useful and the quicker flow of fluid can be used where analyte interactions aren't used.

In some examples the reaction chambers 105 can comprise internal structures can be configured to enable fluid to flow through the reaction chamber 105 by a wicking action.

In this example the reaction chambers 105 comprise meandering structures. This can increase the length of the reaction chamber 105 across the surface of the apparatus 101. This can increase the exposure of the fluid sample to the reagents. Other configurations for the reaction chambers 105 can be used in other examples of the disclosure.

The reaction chambers 105 can be formed from any suitable material. The reaction chambers 105 can be formed from a water impermeable material. The reaction chambers 105 can also be formed from a material that does not react with the reagents or the analytes within the fluid sample. In some examples the reaction chambers 105 can be formed from a polymeric material such as Polydimethylsiloxane (PDMS). Other types of material can be used in other examples.

The reaction chambers 105 can be formed using any suitable process. For instance, if the reaction chambers 105 are formed using a polymeric material, then the reaction chambers 105 can be formed into the desired shapes and dimensions using cast molding or any other suitable process. The cast molding process can comprise photolithographically imprinting the design of the reaction chambers 105 onto a sacrificial substrate to form a molding. The polymeric material such as PDMS is then poured into the template to obtain the reaction chambers 105 or parts of the reaction chambers. In some examples two different moldings can be used to form different parts of the reaction chambers 105. The two different parts can then be sealed together to form a sealed enclosure for the reaction chambers 105.

In this example the apparatus 101 comprises an aperture 701 for receiving an electrode. The electrode can provide a sensor 107 for sensing the presence of an analyte in a fluid sample.

The aperture 701 for receiving the electrode is positioned adjacent to the reaction chambers 105 so that when an electrode is inserted into the aperture 701 the electrode forms, at least part of a wall of the reaction chambers 105. The aperture 701 and the reaction chambers 105 can be configured so that when an electrode is inserted into the aperture 701 the electrode and the reaction chambers 105 form a sealed, or substantially sealed, enclosure.

The electrode that is to be inserted into the apertures 701 can comprise a functionalized electrode. The electrode can be functionalized with any suitable reagent. The reagent that used to functionalize the electrode can be determined by the type of fluid sample and the analyte within the sample that the apparatus 101 is intended to detect and/or by any other suitable factor.

The aperture 701 can be configured to enable a user to insert and remove electrodes from the aperture 701. This can enable the apparatus 101 to be provided as a modular system which enables the user to select which electrode to use. For example, this could enable the user to select which electrode to use based on the sample that they intend to analyse. In such cases the user could select an electrode that has been functionalized with a particular reagent so as to enable the analyte of interest to be detected. This can also enable the apparatus 101 to be re-used so as to enable a plurality of different fluid samples to be analysed.

In other examples the apparatus 101 can be configured so that the electrode would be fixed inside the aperture 701. In such examples the electrode need not be user replaceable and the apparatus 101 could be provided as a single unit.

The apparatus 101 can also be configured with components that are not shown in Fig. 7. For example, the apparatus 101 could comprise one or more temperature sensors and other temperature control means 103. Other arrangements and configuration for the elongated microfluidic channels could be used in other examples. For instance, in some examples something other than a functionalized electrode could be used as the sensor 107. In such examples a thermometer, or any other suitable means could be used as the sensor 107 and the microfluidic channels could be formed so as to enable the sensor 107 to detect the presence and/or concentration of the analytes.

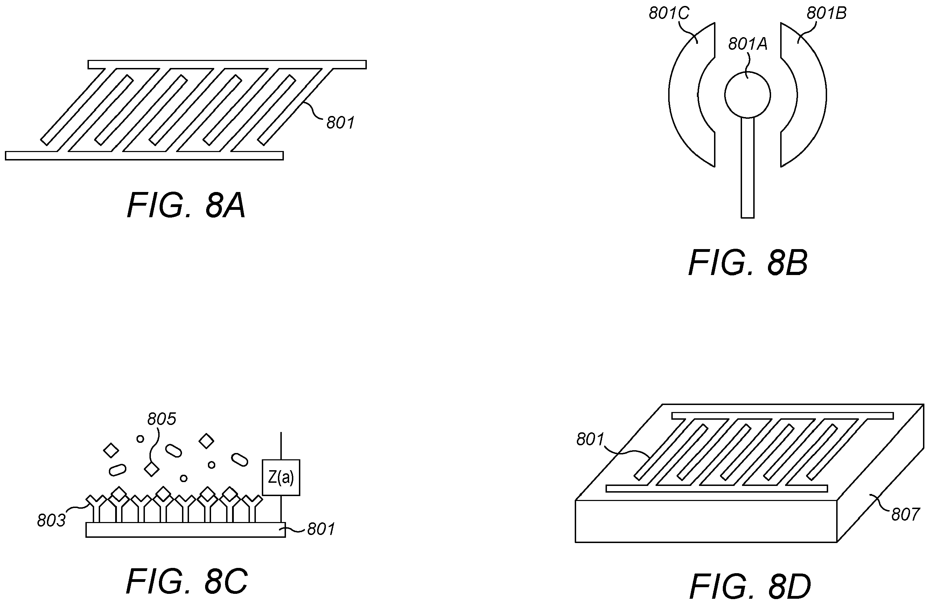

Figs. 8A to 8D shows example electrodes 801 that could be inserted into the aperture 701 of the apparatus 101. Fig. 8A shows an electrode design comprising, two electrodes, Fig. 8B shows an electrode design comprising three electrodes 801, Fig. 8C shows functionalized electrodes 801 and Fig. 8D shows example electrodes 801 on a substrate 807.

The electrodes 801 can be configured to provide sensors 107 for the apparatus 101. The sensors 107 detect the presence of the analyte within the sample. The sensors 107 also provide an output indicative of the presence and/or concentration of the analyte within the sample. The sensors 107 can provide an output indicative of the concentration of the analyte within the sample.

The electrodes 801 can comprise planar electrodes. The electrodes 801 can comprise very thin electrodes 801 that can be formed using chemical vapour deposition methods or any other suitable methods.

The electrodes 801 can be formed from any suitable electrically conductive material such as gold, platinum or graphene. The material that is used can be selected to provide stability during the reactions and also to provide good electrical conductivity. In some examples the electrodes 801 can also be configured as part of the heat transfer means and so the material used can be selected to provide good thermal conductivity.

In the example of Fig. 8A two electrodes 801 are provided in an interdigitated arrangement. The interdigitated arrangement can be configured to maximize, or substantially maximize, contact area between the electrode and a sample fluid in the reaction chambers 105. Other arrangements of two electrodes 801 could be used in other examples of the disclosure, for example the electrodes 801 could comprise intertwined circles or any other suitable shapes.

The arrangement of two electrodes 801 as shown in Fig. 8A can be configured to enable electrical impedance spectroscopy analysis to be performed, or for any other suitable type of analysis.

In the example of Fig. 8B three electrodes 801 are provided. The configuration of Fig. 8B comprises a central electrode 801A, a first electrode 801B on the right-hand side of the central electrode 801A and a second electrode 801C on the left-hand side of the central electrode 801A. The configuration of three electrodes 801A, 801B, 801C can enable different types of analysis to be performed by the apparatus 101. For example, it could enable chemical impedance spectroscopy or other type of analysis.

Fig. 8C schematically shows how an electrode 801 can functionalized. In this example the surface of the electrode 801 is coated with a biomarker 803, or other molecule for binding with analytes 805 in the fluid sample. Any suitable process can be used to attach the biomarker 803 to the electrodes 801. The biomarker 803 can be selected to interact with a specific type of analyte 805 within the fluid samples. Different biomarkers 803 could be used depending upon the type of analyte 805 that is to be detected. As the fluid sample passes over the functionalized electrode 801 the analytes 805 within the sample bind to the biomarker 803. This changes the electrical properties of the electrodes 801 and so enables the electrode 801 to provide an output signal dependent upon the amount of interaction between analytes 805 in the sample and the functionalized electrode 801. The output signal therefore provides an indication of the amount of analyte 805 within the sample.

Fig. 8D shows example electrodes 801 on a substrate 807. The electrodes 801 can be printed onto the substrate 807 or formed by any other suitable process. The substrate 807 is sized and shaped so as to fit into the aperture 701 of an apparatus 101. In the example of Fig. 8D the substrate 807 is a substantially flat substrate 807. Other shapes of substrate 807 can be used in other examples of the disclosure.

The material used for the substrate 807 can be electrically insulating and can be selected so that it does not interact with the analytes 805 or the fluid sample. In some examples the material used for the substrate 807 can be the same as the material used for the apparatus 101. The substrate 807 can comprise PDMS or any other suitable material.

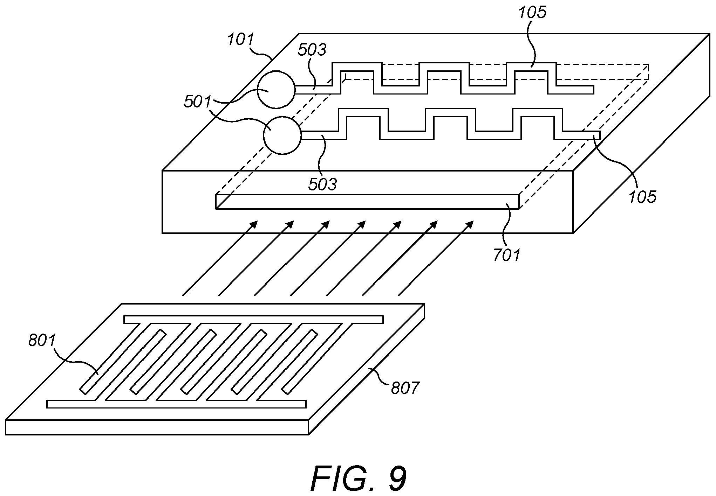

Fig. 9 shows an example electrode 801 and apparatus 101. The electrode is printed on a substrate 807 and is being inserted into the aperture 701 of the apparatus 101 as indicated by the arrows.

Once the electrode 801 is inserted into the aperture the electrode 801 and the substrate 807 form part of the walls of the reaction chambers 105. The electrodes 801 and the substate 807 can seal the reaction chambers 105 so that a fluid sample can flow through the reaction chamber 105.

In some examples the apparatus 101 can comprise means for securing the electrode 801 in position once the electrode 801 and the substrate 807 have been inserted into the aperture 701. In some examples the means could comprise an external hinge that is configured to provide a compressive force to at least part of the apparatus 101. This helps to hold the electrode 801 and the substrate 807 in place within the aperture 701 and can help to seal the reaction chambers 105. In some examples the electrode 801 and the substrate 807 can be made from the same material as the apparatus 101. This can provide a natural adhesion between the substrate 807 and the apparatus 101 and helps to hold the substrate 807 in place within the aperture 701.

Once the reaction has been completed the electrode 801 and the substrate 807 can be removed from the aperture 701. In some examples the electrode 801 and substrate 807 could be replaced with a different electrode 801 and substrate 807. This can enable the apparatus 101 to be reused to test a plurality of different samples.

In some examples the apparatus 101 could be cleaned after it has been used. For instance, once the electrode 801 and the substrate 807 have been removed a cleaning fluid can be passed through the reaction chambers 105.

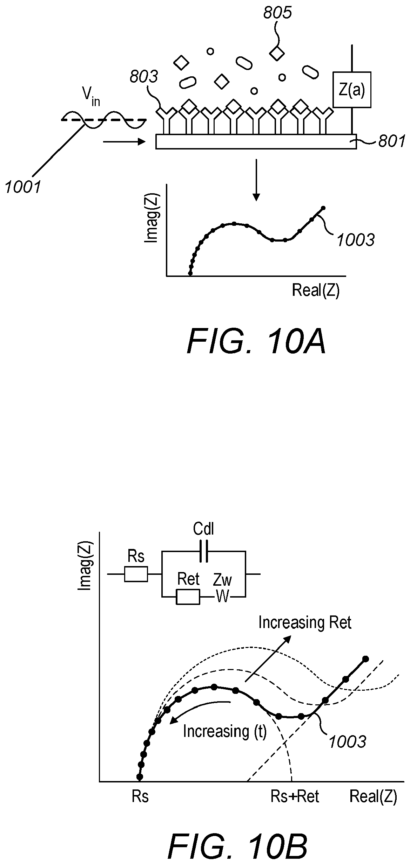

Figs. 10A and 10B shows an example output electrode 801 in use. Fig. 10A shows an example input signal 1001 and an example output signal 1003. Fig. 10B shows how an output signal 1003 could change depending upon concentration of an analyte within a fluid sample.

The electrodes 801 and the input signal 1001 can be configured for any suitable type of analysis of a sample. For example, if the analysis could comprise Electrochemical Impedance Spectroscopy (ECIS), electrical impedance spectroscopy (EIS), lon-Sensitive Field Effect Transistor (ISFET) or any other suitable type of analysis.

In the example of Fig. 10A the electrode 801 is a functionalized electrode 801. The electrode 801 has been functionalized with a biomarker 803 that has been selected to bind with the analyte 805 of interest. When the electrode 801 is exposed to a fluid sample the analyte of interest will bind to the biomarker 803.

When the electrode 801 is in use an input signal 1001 is provided to the electrode 801. The input signal 1001 can be provided to the electrode 801 via a custom-built adapter or via any other suitable electrical connection.

In the example of Fig. 10A the input signal 1001 comprises a sinusoidal alternating current signal. Other types of signals could be used in other examples of the disclosure. The input signal 1001 can be selected to have predetermined electrical parameters such as frequency, amplitude or cycle. The parameters could be selected based on the electrodes 801, the analyte 805 that is to be detected, the biomarkers that have been used and/or any other suitable factor.

The input signal 1001 passes through the electrode 801 and provides an output signal 1003. The electrical properties of the electrode 801 will be dependent upon the amount of analyte 805 that has interacted with the biomarker 803. The electrical properties of the electrode 801 will affect the output signal 1003 that is provided. Therefore, the output signal 1003 provides an indication of how the analyte 805 that has interacted with the biomarker 803 and so provides an indication of the amount of analyte 805 within a sample.

The electrode 801 therefore provides a sensor 107 that can sense an analyte within a sample.

In some examples a reference output signal can be obtained from the electrode 801 before it is exposed to a sample. The reference output signal can provide a default measurement of impedance, phase reactance and/or other any other suitable property of the electrode 801. Once the electrode 801 has been exposed to a fluid sample the change in the properties of the output signal can be used to determine the concentration of the analyte 805 within the fluid sample. Fig. 10B shows an example of how the properties of an output signal could change. Therefore, by comparing the output signal 1003 to a reference output signal an indication of the concentration of an analyte within a sample could be obtained.

Examples of the disclosure therefore provide an apparatus 101 that can be inserted into a user's electronic device 115 to enable analysis of fluid samples. This can provide a cost effective and convenient means for analysing fluid samples. By enabling heat from the electronic device to be provided to the apparatus 10 comprising the sample this can enable the apparatus 101 to be used for a wide range of analytes and reactions. For example, the apparatus could be used for PCR reactions which require specific temperature conditions.

In this description the term coupled means operationally coupled. Any number or combination of intervening elements can exist between coupled components including no intervening elements.

The term 'comprise' is used in this document with an inclusive not an exclusive meaning. That is any reference to X comprising Y indicates that X may comprise only one Y or may comprise more than one Y. If it is intended to use 'comprise' with an exclusive meaning, then it will be made clear in the context by referring to "comprising only one..." or by using "consisting".

In this description, reference has been made to various examples. The description of features or functions in relation to an example indicates that those features or functions are present in that example. The use of the term 'example' or 'for example' or 'can' or 'may' in the text denotes, whether explicitly stated or not, that such features or functions are present in at least the described example, whether described as an example or not, and that they can be, but are not necessarily, present in some of or all other examples. Thus 'example', 'for example', 'can' or 'may' refers to a particular instance in a class of examples. A property of the instance can be a property of only that instance or a property of the class or a property of a sub-class of the class that includes some but not all of the instances in the class. It is therefore implicitly disclosed that a feature described with reference to one example but not with reference to another example, can where possible be used in that other example as part of a working combination but does not necessarily have to be used in that other example.

Although examples have been described in the preceding paragraphs with reference to various examples, it should be appreciated that modifications to the examples given can be made without departing from the scope of the claims.

Features described in the preceding description may be used in combinations other than the combinations explicitly described above.

Although functions have been described with reference to certain features, those functions may be performable by other features whether described or not.

Although features have been described with reference to certain examples, those features may also be present in other examples whether described or not.

The term 'a' or 'the' is used in this document with an inclusive not an exclusive meaning. That is any reference to X comprising a/the Y indicates that X may comprise only one Y or may comprise more than one Y unless the context clearly indicates the contrary. If it is intended to use 'a' or 'the' with an exclusive meaning then it will be made clear in the context. In some circumstances the use of 'at least one' or 'one or more' may be used to emphasis an inclusive meaning but the absence of these terms should not be taken to infer any exclusive meaning.

The presence of a feature (or combination of features) in a claim is a reference to that feature or (combination of features) itself and also to features that achieve substantially the same technical effect (equivalent features). The equivalent features include, for example, features that are variants and achieve substantially the same result in substantially the same way. The equivalent features include, for example, features that perform substantially the same function, in substantially the same way to achieve substantially the same result.

In this description, reference has been made to various examples using adjectives or adjectival phrases to describe characteristics of the examples. Such a description of a characteristic in relation to an example indicates that the characteristic is present in some examples exactly as described and is present in other examples substantially as described.

Whilst endeavoring in the foregoing specification to draw attention to those features believed to be of importance it should be understood that the Applicant may seek protection via the claims in respect of any patentable feature or combination of features hereinbefore referred to and/or shown in the drawings whether or not emphasis has been placed thereon.

## Claims

1. An apparatus:
wherein the apparatus is configured to be removably inserted into an input port of an electronic device and wherein the apparatus comprises:
at least one reaction chamber configured to receive a fluid sample and enable the fluid sample to come into contact with one or more reagents;
at least one sensor for sensing a reaction between the fluid sample and the one or more reagents; and
temperature control means configured to use heat from the electronic device to control a temperature of the fluid sample in the at least one reaction chamber.

2. An apparatus as claimed in claim 1 wherein the at least one sensor for sensing a reaction comprises at least one functionalized electrode.

3. An apparatus as claimed in claim 2 wherein the at least one functionalized electrode is configured to be user replaceable.

4. An apparatus as claimed in any preceding claim wherein the temperature control means comprises one or more sensors for determining the temperature of the at least one reaction chamber and means for providing a control signal to control heat provided from the electronic device to the at least one reaction chamber based upon the determined temperature.

5. An apparatus as claimed in any preceding claim comprising means for providing an output indicative of the presence and concentration of an analyte within the fluid sample.

6. An apparatus as claimed in any preceding claim comprising one or more data connections configured to enable data to be transmitted from the apparatus to the electronic device.

7. An apparatus as claimed in any preceding claim comprising means for receiving the fluid sample and means for enabling the fluid sample to flow from the means for receiving the fluid sample into at least one reaction chamber.

8. An apparatus as claimed in any preceding claim wherein the at least one reaction chamber comprises at least one elongated microfluidic channel and one or more functionalized electrodes.

9. An electronic device comprising:
one or more heat sources;
one or more input ports configured to enable an apparatus for analysing a fluid sample to be removably inserted into the input port; and
heat transfer means configured to transfer heat from the one or more heat sources to a location proximate to the one or more input ports.

10. An electronic device as claimed in claim 9 wherein the heat transfer means comprises at least one of: a heat pipe, thermosyphon loop, oscillating heat pipe.

11. An electronic device as claimed in any of claims 9 to 10 wherein the heat transfer means comprise a thermal interface in the one or more input ports configured to enable heat to be transferred to an apparatus for analysing a fluid sample when the apparatus for analysing a fluid sample is removably inserted into the input port.

12. An electronic device as claimed in any of claims 9 to 11 comprising one or more data connections configured to enable data to be transmitted between the electronic device and the apparatus for analysing a fluid sample.

13. An electronic device as claimed in any of claims 9 to 12 wherein the one or more heat sources comprise at least one of: a battery, electronic components, optoelectronic components, processors.

14. An electronic device as claimed in any of claims 9 to 13 wherein at least one of the one or more input ports is configured to receive a plurality of different input components.

15. An electronic device as claimed in any of claims 9 to 14 wherein the electronic device comprises a hand-held communications device.
